# EUROPEAN PATENT APPLICATION

(11) **EP 1 902 739 A1**
(43) Date of publication of application: **26.03.2008**
(21) Application number: 06291473.4
(22) Date of filing: 20.09.2006
(51) Int. Cl.: A61L 27/24, A61L 27/38, A61L 27/50

(54) **Synthetic multi-layer structures comprising biopolymer fibres**

(71) Applicant: CENTRE NATIONAL DE LA RECHERCHE SCIENTIFIQUE (CNRS), 75016 Paris (FR)
(72) Inventor: Torbet, James, 38330 St. Ismier (FR); Hulmes, David John Stuart, 69230 Saint Genis Laval (FR)
(74) Representative: Warcoin, Jacques

(57) **Abstract**

The invention relates to a method for the *in vitro* preparation of a synthetic multi-layer structure comprising biopolymer fibers, wherein the biopolymer fibers in each layer are unidirectionally oriented, which comprises successive polymerisation of layers of a biopolymer fiber forming solution in the presence of a magnetic field. Advantageously, the fiber orientation in at least one layer differs from that in at least one of its superior and/or inferior layer according to an angle alpha. The invention also relates to a biological tissue-like multi-layer structure comprising the synthetic multi-layer structure and cells inoculated therein, such as an orthogonal multi-layer collagen tissue-like cornea, and the method for its preparation. The invention may be used for preventing or treating a damaged tissue, or for creating a model for biological testing, such as pharmacotoxicity testing.

## Description

The invention relates to a method for the *in vitro* preparation of a synthetic multi-layer structure comprising biopolymer fibers, wherein the biopolymer fibers in each layer are unidirectionally oriented, which comprises successive polymerisation of layers of a biopolymer fiber forming solution in the presence of a magnetic field. Advantageously, the fiber orientation in at least one layer differs from that in at least one of its superior and/or inferior layer according to an angle alpha. The invention also relates to a biological tissue-like multi-layer structure comprising the synthetic multi-layer structure and cells inoculated therein, such as an orthogonal multi-layer collagen tissue-like cornea, and the method for its preparation. The invention may be used for preventing or treating a damaged tissue, or for creating a model for biological testing, such as pharmacotoxicity testing.

The world market for tissue engineering products is estimated to several hundreds of million euros. Tissue engineering seeks to repair, replace or restore tissue function, typically by combining biomaterials and living cells.

More specifically, around 10 million people in the world suffer from blindness due to lesions or diseases of the cornea. The present world market for the artificial corneas is estimated to approximately 100,000 units per year, including 25,000 in Europe. Since the present sale price of an artificial corneal implant amounts to approximately 2000 euros, the market is estimated to around 120 million euros.

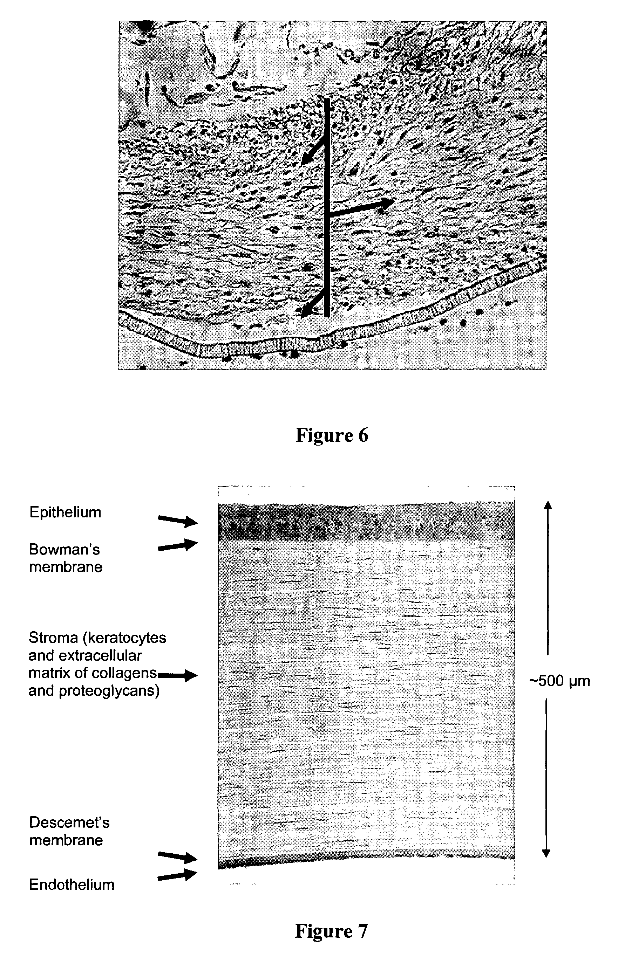

The cornea consists of three distinct cellular layers: the outer epithelium, the central stroma (composed of keratocytes embedded in a dense, highly organized extracellular matrix of collagen fibrils and proteoglycans) and inner endothelium The stroma is delimited by specialized acellular structures, Bowman's and Descemet's membranes, lying at the interface between the epithelium and endothelium respectively (see Figure 7).

The stroma makes up some 90% of the corneal volume and 70% of the cornea dry weight. During embryogenesis a highly structured acellular primary stroma forms which is believed to dictate the pattern of organization of the adult stroma (Trelstad and Coulombre, 1971a). Within adult stroma the collagen fibrils, composed of collagens types I and V, are of uniformly narrow (≈30 nm) diameter and are arranged in lamellae, within which fibrils are parallel and separated by a proteoglycan rich matrix. The collagen fibrils are stabilized by intra- and inter-molecular covalent crosslinks, which confer tensile strength and stabilize fibrils against proteolytic degradation. Transparency is believed to be largely dependent upon the ordered three-dimensional architecture of thin collagen fibrils. Electron microscopy (Trelstad and Coulombre, 1971c) and x-ray diffraction (Meek et al., 1987) studies indicate that fibrils in adjacent stromal lamellae are predominantly orthogonal. The interlacing of stromal lamellae endows the cornea with highly non-linear biomechanical properties - becoming increasingly stiffer under high intra-ocular pressure which enables the cornea to survive abnormal influences such as impact, injury and surgery without bursting.

The construction of a scaffold that closely reproduces native stroma architecture including orthogonal lamellae consisting of aligned collagen fibrils, would be an important asset in the development of bio-engineered corneal implants. In view of the world-wide shortage of donors, the increasing risk of transmissible diseases, the widespread use of corrective surgery that renders corneas unsuitable for grafting, and the severe limitations of currently available synthetic polymer-based artificial corneas (keratoprostheses), there is an urgent need to develop new therapeutic strategies.

Recent advances in tissue engineering, stem cell technology and nano-engineering have been successfully used to repair human corneas and even recreate a human cornea-like tissue de novo. Grafts of human cornea-limbal epithelial sheets, cultured on a fibrin, show long-term clinical efficacy in the regeneration of damaged corneal surface (Rama et al., 2001;Pellegrini et al., 1997). Damaged eye tissue has also been restored using epithelial cells cultured on amniotic membranes (Tsai et al., 2000;Schwab et al., 2000) and temperature-responsive cell culture surfaces (Nishida et al., 2004).

Stand alone acellular matrices with some stromal-like properties, supporting cell penetration and tissue regeneration once implanted, have also been developed with some encouraging results (Li et al., 2005;Liu et al., 2006). The latter approach amounts to providing an optimised scaffold to facilitate cell colonisation and tissue remodelling following implantation. If successful this tactic would have the virtue of being easier and cheaper to implement in a clinical setting than more complex solutions.

However, all the biomatrices so far created totally lack the highly organized three-dimensional collagen-based architecture present in native stroma.

Collagen has been successfully aligned using a number of techniques. Hydrodynamic flow or electrospinning can generate oriented collagenous sheets. The former gives rise to ultrathin ribbons of highly oriented small diameter (~3 nm) fibrils (Jiang et al., 2004). While electrospinning results in less well oriented thicker fibers of variable diameter (Boland et al., 2004;Matthews et al., 2002). However, it has not been demonstrated that either of these techniques can give rise to anything other than uni-directionally aligned materials. Another approach exploits the spontaneous liquid crystalline ordering that takes place with time in collagen solutions at high concentration. A cholesteric phase forms in which the molecules are aligned in planes that continuously rotate in a helical manner. Dense three-dimensionally ordered matrices are formed by inducing fibrillogenesis (Besseau et al., 2002;Mosser et al., 2006). The colonization of these dense collagen (≤40 mg/ml) matrices by human dermal fibroblast to a depth of 400µm within a month demonstrates that they have potential tissue engineering applications.

Highly oriented collagen (Torbet and Ronziere, 1984;Murthy, 1984) is produced when a solution of molecules is transformed into a gel in a strong magnetic field because of the cumulative effect of the many weakly diamagnetically anisotropic molecules (Worcester, 1978). Many cell types, including fibroblasts (Guido and Tranquillo, 1993), keratinocytes, osteoblasts (Kotani et al., 2000), neurites (Dubey et al., 1999), endothelial cells undergoing angiogenesis (Torbet et al., 2000) are aligned by contact guidance when seeded on these magnetically oriented collagen or fibrin substrates. Two tissue engineering applications based on the magnetic orientation of these biopolymers have been proposed: hollow tubes having an outer wall of circumferentially oriented type I collagen for bioartificial arteries (Tranquillo et al., 1996) and rods with uni-axially aligned fibrin or collagen for nerve regeneration (Ceballos et al., 1999).

There is a need for developing tissue-like multi-layer structures which may be used as implants in medicine. Moreover, such tissue-like multi-layer structures should conform the anatomy of natural tissues, such as orthogonal structure of the cornea.

The present Inventors have solved this problem by developing a process which comprises successive polymerisation of layers of a biopolymer fiber forming solution in the presence of a magnetic field. A lamellar scaffold is thus obtained, comprising fibers which are unidirectionally oriented within each layer, resembling those present in vivo. This solution offers the great advantage that the layers of oriented polymerised fibers are not altered by subsequent applications of the magnetic field, and thus keep their orientation unchanged.

Moreover, the lamellar scaffold of the present invention may comprise a differential orientation of the biopolymer fibers from one layer to the next one. Thus, laminated corneal stroma-like scaffolds consisting of multiple intermeshed orthogonal layers of oriented collagen fibers may be created. Keratocytes seeded on unidirectionally oriented collagen gels become uniformly oriented along the direction of the collagen fibrils and penetrate into the collagen gel where they align following the direction of the collagen fibrils within the lamellae. As epithelial and endothelial cells also migrate and proliferate on this scaffold they can form the basis for the reconstruction of hemi or complete cornea equivalents for use in lamellar or penetrating keratoplasty, respectively.

Scaffolds can be used as stand alone implants, or be seeded with cells prior to implantation or implanted after being allowed to mature *in vitro* until tissue-like properties develop. In addition to their potential clinical uses, tissue-engineered human corneal equivalents can also serve as alternatives to animal models for cosmeto-pharmacotoxicity testing. As three-dimensional organ mimics, they can also facilitate the *in vitro* study of the complex physiology of living tissue.

Thus, in a first aspect, the invention is aimed at a method for the *in vitro* preparation of a synthetic multi-layer structure comprising or consisting of biopolymer fibers, wherein the biopolymer fibers in each layer are unidirectionally oriented, which comprises the following steps :
a) positioning a holder containing a layer of a biopolymer fiber forming solution in a magnetic field and inducing fiber formation, in order to obtain fibers which are unidirectionally oriented in the layer,
b) introducing a further layer of the biopolymer fiber forming solution in the holder, said further layer being introduced over the layer obtained in step a),
c) positioning the holder in the magnetic field and inducing fiber formation, in order to obtain fibers which are unidirectionally oriented in said further layer,
d) optionally, repeating the preceding steps,
thus obtaining a synthetic multi-layer structure.

The expression "multi-layer" refers to a structure comprising two or more layers.

The expression "oriented" or "unidirectionally oriented" refers herein to biopolymer fibers which are substantially aligned in a specific direction. This orientation can be checked using birefringence and/or electronic microscopy and/or optical microscopy.

The holder refers to any container which is appropriate for use in the present invention, in particular for allowing the biopolymer to orient upon application of the magnetic field. It may be of any shape, such as a well having a flat base, or a mold, such as a cylindrical mold having an annular cross-section. The cylindrical mold can be positioned vertically in a horizontal magnetic field. Such cylindrical molds having an annular cross-section find an application for preparing tissue-like compact bones or intervertebral discs. The well having a flat base can be positioned horizontally in a horizontal magnetic field. Wells are preferably used for preparing tissue-like corneas.
Advantageously, the holder has an impermeable or semi-permeable base.

In a preferred embodiment, the fiber orientation in at least one layer differs from that in at least one of its superior and/or inferior layer according to an angle alpha, and said differential orientation results from changing by a rotation according to the angle alpha the position of the holder of step c) relative to the position of the holder of step a).

In a preferred embodiment, the fiber orientation in each layer differs from that in at least one of its superior and/or inferior layer according to the angle alpha.

Biopolymers suitable for use in the present invention include any polymer which is in the form of a solution in certain conditions, and which can form fibers by modifying said conditions. The method of the invention also requires that the biopolymers orient in a unique direction upon the application of a magnetic field. Advantageously, the biopolymer fibers are collagen fibers, fibrin fibers, or a mixture thereof. More preferably, the collagen fibers are type I and/or type V collagen fibers, and/or any other fiber forming collagen.

The thickness of the layers of the biopolymer fiber forming solution in the holder is advantageously less than 3 mm.

It is well known in the art that the collagen fiber forming solution is acid and cold, and that collagen fibers are obtained by raising the pH and ionic strength of acid-soluble collagen as well as by heat precipitation of neutral salt-soluble collagen (see for example Murthy, 1984). In a preferred embodiment, the collagen fiber formation is thus induced by neutralising and/or heating the collagen fiber forming solution, advantageously to a pH ranging from 7 to 8 and/or to a temperature ranging from 28°C to 35°C, advantageously 30°C.

As it is well known in the art, conditions for inducing fibrin fiber formation are different from that of collagen, since fibrinogen is used to obtain fibrin fibers, by the action of the enzyme thrombin. Likewise, it is also possible to induce collagen fiber formation by proteolytic processing of soluble procollagen precursors, using specific enzymes. Such methods of inducing fiber formation could also be used to obtain multi-layer structures using the methodology described herein.

Advantageously, the concentration of the bioploymer fiber forming solution ranges from 1 to 10 mg/mL.

More preferably, the neutralisation of the solution is made prior to the heating step, thus allowing to introduce the neutralised biopolymer fiber forming solution in the holder to form one layer, and the heating step is made in the presence of the magnetic field, in order to obtain fibers which are unidirectionally oriented in the layer.

The physical and/or biological properties of the synthetic multi-layer structure may be changed by adding compounds, preferably biocompatible compounds. For example, proteoglycans in solution may be added for improving optical transparency of the synthetic multi-layer structure. Thus, advantageously, the synthetic multi-layer structure further comprises proteoglycans. More advantageously, said proteoglycans are added before fiber formation. Most advantageously, said proteoglycans are added to the neutralised biopolymer fiber forming solution.

Advantageously, covalent cross-linkages are introduced in order to confer tensile strength and stabilize fibers against proteolytic degradation. Introduction is advantageously made to the obtained synthetic multi-layer structure.

Thus, in another embodiment, the synthetic multi-layer structure further comprises covalent cross-linkages obtained using chemical compounds such as 1-ethyl-3-(3-dimethylaminopropyl) carbodiimide hydrochloride (EDC) or N-hydroxysuccinimide ester (NHS).

In another embodiment, the synthetic multi-layer structure further comprises covalent cross-linkages obtained using enzymes such as transglutaminase or lysyl oxidase.

The biopolymer fibers to be used in the invention may result from an extract of a biological tissue, or may be recombinant. Preferably, the biopolymer fibers are recombinant biopolymer fibers, advantagesouly human recombinant biopolymer fibers. Recombinant biopolymer fibers may be obtained from FIBROGEN Inc.

Advantageously, the synthetic multi-layer structure is dehydrated by evaporation or blotting using a semi-permeable support. Dehydratation allows to concentrate the synthetic multi-layer structure in order to obtain a structure which resembles those present *in vivo.* Advantageously, the concentration of the synthetic multi-layer structure ranges from 100 mg/mL to 200 mg/mL, advantageously 160 mg/mL.

More advantageously, the synthetic multi-layer structure is lyophilized. Lyophilized synthetic multi-layer structures of the invention are very appropriate for storage. Lyophilization further allows to render the structure more porous.

The appropriate magnetic field for use in the present invention is generally a high strength (4.7 to 9 Tesla) magnetic field. Preferably, the magnetic field is a static magnetic field, and may be generated by a horizontal bore electromagnet, such as those from the Oxford Company of the United Kingdom.

Preferably, the angle alpha is comprised between 30° and 150°. In a particularly advantageous embodiment, the angle alpha is about 90°, thus obtaining a synthetic orthogonal multi-layer structure.

In a second aspect, the invention is aimed at a synthetic multi-layer structure comprising or consisting of biopolymer fibers, wherein the biopolymer fibers in each layer are unidirectionally oriented, said structure being obtainable by the method for the *in vitro* preparation of a synthetic multi-layer structure according to the invention.

In an advantageous embodiment, the fiber orientation in at least one layer, preferably each layer, differs from that in at least one of its superior and/or inferior layer according to the angle alpha.

Preferably, the biopolymer fibers are collagen fibers, fibrin fibers, or a mixture thereof. More preferably, the collagen fibers are type I and/or type V collagen fibers.

In another embodiment, the synthetic multi-layer structure further comprises proteoglycans.

In a further embodiment, the synthetic multi-layer structure further comprises covalent cross-linkages obtained using chemical compounds such as 1-ethyl-3-(3-dimethylaminopropyl) carbodiimide hydrochloride (EDC) or N-hydroxysuccinimide ester (NHS).

In another embodiment, the synthetic multi-layer structure further comprises covalent cross-linkages obtained using enzymes such as transglutaminase or lysyl oxidase.

Advantageously, the biopolymer fibers are recombinant biopolymer fibers, advantagesouly human recombinant biopolymer fibers.

In a particular advantageous embodiment, the synthetic multi-layer structure according to the invention is lyophilized.

Preferably, the angle alpha is comprised between 30° and 150°. More preferably, the angle alpha is 90°, and the structure is a synthetic orthogonal multi-layer structure.

In a third aspect, the invention is directed to a method for the *in vitro* preparation of a biological tissue-like multi-layer structure which comprises the preparation of a synthetic multi-layer structure according to the method of the present invention, and the inoculation of cells therein.

The cells to be inoculated may be of any type. The cell type is generally that of the tissue which it is intended to re-create, for example smooth muscle cells for a muscle-like tissue. Preferably, the cells are mammalian cells, more preferably human cells.

In a preferred embodiment, the biological tissue-like multi-layer structure is a multi-layer collagen tissue-like structure.

More preferably, the multi-layer collagen tissue-like structure is a multi-layer collagen tissue-like cornea, such as corneal stroma, hemi-cornea or complete cornea. Preferably, the cells are keratocytes, endothelial cells, epithelial cells or a combination thereof.

Cells may be added to the biopolymer fiber forming solution prior to assembly of the fibers and following adjustment to near physiological conditions of pH and ionic strength. Cells may also be added to the previously formed multi-layer structure. Thus, in a preferred embodiment, the cell inoculation is made by adding the cells to the neutralised collagen fiber forming solution, and/or by adding the cells to the multi-layer collagen tissue-like structure.

In a further aspect, the invention is aimed at a biological tissue-like multi-layer structure comprising the synthetic multi-layer structure according to the invention and cells inoculated therein. Said biological tissue-like multi-layer structure may be obtained by the method as described above.

Preferably, the cells are mammalian cells, advantageously human cells.

In a preferred embodiment, the biological tissue-like multi-layer structure according to the invention is a multi-layer collagen tissue-like structure, advantageously an orthogonal multi-layer collagen tissue-like cornea, such as a corneal stroma, a hemi-cornea or a complete cornea. Preferably, the cells are keratocytes, endothelial cells, epithelial cells or a combination thereof.

The biological tissue-like multi-layer structure may further resemble any tissue having a multi-layer structure, such as those where the direction alternates from one layer to the next, including, for example, in addition to cornea, compact bone or intervertebral disc.

In a further aspect, the invention is aimed at the use of the synthetic multi-layer structure according to the invention for the preparation of a biological tissue-like multi-layer structure.

Moreover, the present invention is aimed at the biological tissue-like multi-layer structure according to the invention for use as a medicament.

In a further aspect, the invention is aimed at the use of the biological tissue-like multi-layer structure according to the invention for the preparation of a medicament for preventing or treating a damaged biological tissue in a subject, such as a mammal, preferably a human.

Also comprised herein is a method of treating and/or preventing a damaged biological tissue in a subject using the biological tissue-like multi-layer structure according to the invention.

Moreover, the present invention is aimed at the use of the biological tissue-like multi-layer structure according to the invention as a model for biological testing, such as pharmacotoxicity testing.

In a further aspect, the invention is directed to the use of the orthogonal multi-layer collagen tissue-like cornea according to the invention for the preparation of a medicament for preventing or treating a damaged corneal tissue in a subject, such as corneal stroma, hemi-cornea, complete cornea or a combination thereof. Preferably, the cells are keratocytes, endothelial cells, epithelial cells or a combination thereof.

Also comprised herein is a method of treating and/or preventing a damaged corneal tissue in a subject using the orthogonal multi-layer collagen tissue-like cornea according to the invention.

The invention is further embodied in the following examples and figures.

### LEGEND OF THE FIGURES

**Figure 1** Change in turbidity as acid soluble rat tail type I (c=1.84 mg/ml) collagen assembles into fibrils following neutralisation with buffer.
**Figure 2.** SEM of collagen fibrils A single oriented lamella; B two orthogonal lamellae.
**Figure 3.** Optical micrograph descending vertically through a gel (2 mg/ml) consisting of three lamellae. The middle lamella is perpendicular to the other two. A 90° switch in orientation is also observed going from the middle to the bottom layer.
**Figure 4.** Stained section showing a three lamellae scaffold. The collagen in the middle lamella is oriented horizontally. The three layers are shown my the variation in stain distribution. Total thickness 38 µm. The thickness before drying was ≈600 µm.
**Figure 5.** Keratocytes seeded on unoriented (left) and oriented (right) collagen gels. The cells align with the oriented fibrils (arrows).
**Figure 6.** Keratocytes were seeded onto a three lamellae scaffold. The cells on the top and bottom layers are aligned perpendicular to the surface; the middle layer is oriented horizontally. The cells have infiltrated the matrix and their shape indicates that they are aligned by contact guidance. The semipermeable support is visible at the bottom.
**Figure 7.** Structure of the cornea.

### EXAMPLES

### Materials & Methods

### Oriented 3-D collagen scaffolds:

Acid soluble rat tail type I collagen (0.02N acetic acid; ≈4 mg/ml) from BD Biosciences and bovine skin type I collagen gifted by Symatese biomatériaux were used. This solution was neutralised at 4°C with an equal volume of buffer (60mM phosphate, 60 mM HEPES, 270 mM NaCl, pH=7.4). Aliquots were then pipetted onto the precooled sample holder and positioned horizontally in the central region of the magnet bore which was at 20°C. After a few minutes the temperature was raised progressively to 30°C over a period of about 10 min. The passage in the magnet lasted for a total of about 30 min. During this period the collagen molecules assemble into fibrils and fibres transforming the solution into a viscous gel. In order to create orthogonal lamellae samples that had already undergone orientation were removed from the magnet, and covered with aliquot of cold neutralized collagen solution and replaced in the magnet rotated by the desired angle with respect to the previous orientation. Using this method scaffolds organized in an orthogonal pattern were constructed.

Sample holders were either glass bottomed plastic culture dishes (MatTek Co.) or Transwell® semi-permeable polycarbonate supports (Corning Co.). The culture dishes (outer diameter 35 mm) had a shallow (1.5 mm deep) central glass well (14 mm diameter). Transwell plates were cut to produce individual wells. The wells (12 mm inner diameter) were placed in standard 35 mm plastic petri dishes. The pore diameter of the semi-permeable membrane was 0.4, 3 or 12 µm. The volume of neutralized collagen solution added to the wells varied from 80 to 200 µl which correspond to a fully hydrated thickness of 0.5 to 1.3 mm. The thickness of the gel is controlled by the volume of the initial solution.

A split-coil superconducting magnet (Thor Cryogenics) with horizontal room temperature bore was used. The bore was horizontal, 5 cm diameter and had a total length of 40 cm. A temperature stabilizing jacket reduces the effective diameter to about 4.5 cm. The field profile rose to a flat plateau extending ±5 cm about the bore centre making it possible to simultaneously place up to three 35 mm diameter samples in maximum field. The temperature of the sample space was controlled by circulating water from a temperature controlled bath. The field used for processing was near the maximum possible (7 tesla). This magnet was provided by the Grenoble High Magnetic Field Laboratory funded by the CNRS.

Turbidity, defined as the optical density, was measured at 400 nm using a Beckman spectrophotometer. Solutions were mixed in the cold and transferred to a prewarmed sample cell (1 mm optical path length quartz cells) positioned in the spectrophotometer.
Dehydrated scaffolds could be conserved for several months but once rehydrated with conservation medium they were used within 7 days. The conservation medium was
200 µl of DMEM using double strength antibiotic concentration at +4°C
DMEM 50 ml; Penicilline G 50µL (200 UI/ml); Gentamycin 100µl (40 µg/ml); Fungizone 100µl (2 µg/ml)

Keratocytes were isolated from human corneas removed in accordance with ethical regulations. The corneas were strored at 31 °C in organ culture, but were unusable for treatment because their endothelial density was too low. Isolation was performed with collagenase A (Roche, ref. 10103586), 3 mg/ml for 3 hours at 31 °C with stirring at 200 rpm. The digest was purified through a 70 µm cell sieve (BD Falcon ref. 352350) and immediately placed in monolayer culture.

Keratocytes were seeded at a density of 10,000 cells/cm² then cultured in a specially designed medium providing optimal growth and preservation of the phenotype as followed : DMEM/Ham-F12 1:1, 10% NCS, 5 ng/ml bFGF, antibiotics. The medium was changed every two days until cell confluence was reached. At confluence, cells were resuspended by trypsin-EDTA 0.5g trypsin/1 and 0.2 g EDTA/1 (Sigma ref. CR0303), and amplified over 3 passages (from P0 to P2) and seeded at P3 inside the matrix.
SEL, TEM methods ...

### Results

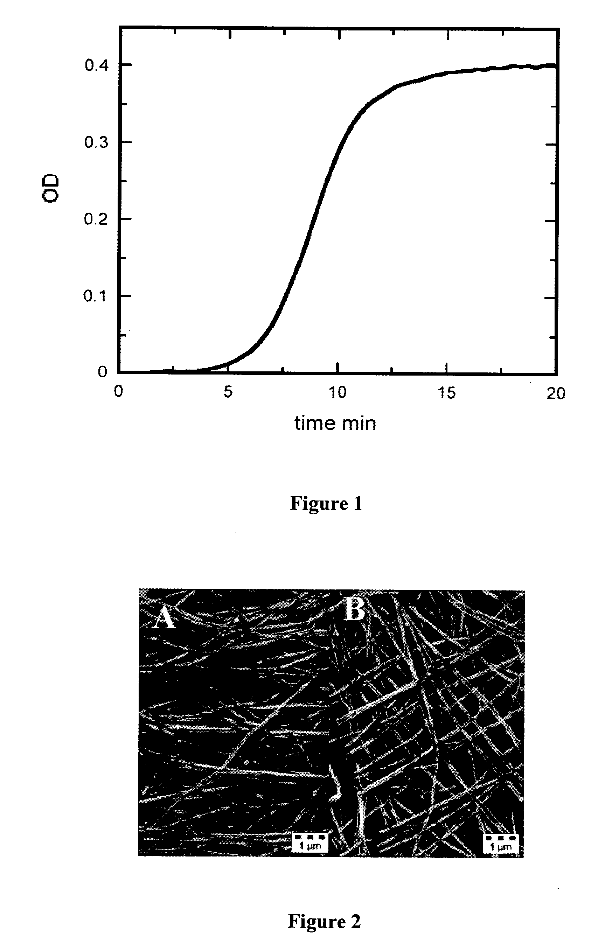

*[Figures 1 & 2A]* A typical type I collagen assembly curve obtained following neutralization of acid soluble collagen with buffer is shown in figure 1. The resulting gel (2mg/ml, 1mm thick) is translucent. When this assembly process takes place in a strong magnetic field an aligned gel is produced (fig. 2A). Although collagen molecules orient perpendicular to the applied field direction due to the negative value of their diamagnetic anisotropy (Worcester, 1978c), high alignment results thanks to the influence of surface constraints (Torbet and Ronziere, 1984a). The fibrils are two to four times thicker (60-110 nm) than those of native stroma and they are also frequently aggregated into large bundles which are generally better oriented than single fibrils. The degree of orientation is sufficient to induce contact guidance of keratocytes as shown below. The preparative procedures required for SEM visualization significantly compact and possibly distort the gel.

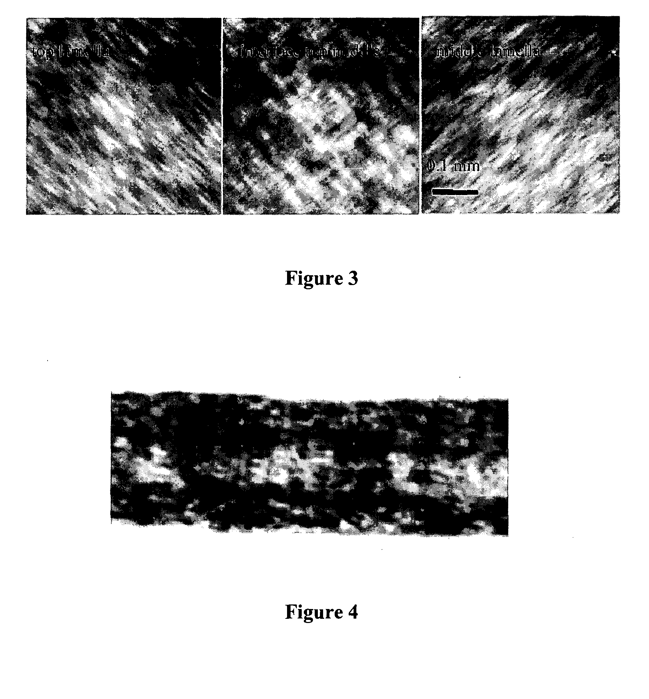

*[Figure 2B, 3 & 4]* As the collagen gels are stable they do not loose orientation on removal from the field so they can be utilized as oriented scaffolds in their initial highly hydrated state or be dehydrated and subsequently rehydrated. Stability also means that multilayers with any desired relative orientation can be created by adopting a layer-by-layer fabrication method. This is achieved simply by covering an already formed gel with a fresh neutralized collagen solution and repeating the assembly process in the magnetic field with the sample rotated with respect to the field direct. By combining a series of gellation-rotation-gellation cycles scaffolds of orthogonal lamellae have been constructed. This method of fabrication also ensures that adjacent layers are intimately interconnected as the added freshly neutralized collagen solution should penetrate the surface layers of the existing gel so forming an interlaced boundary layer resembling native cornea (Fig. 2).

The final concentration of collagen in the oriented gels (≈2 mg/ml) is well below that present in cornea stroma, which is in the region of 160 mg/ml. However, the hydrated scaffolds can be readily concentrated into sheets to any desired degree with little or no loss in orientation either by evaporation or blotting the semipermeable support. Evaporation is slow and results in the build-up of salt and buffer, while blotting is rapid and causes no change in osmotic conditions, consequently it can be used in the presence of cells. The fully hydrated scaffolds have also been lyophilized to provide porous sponges but so far we have not carried out cell growth studies on this support.

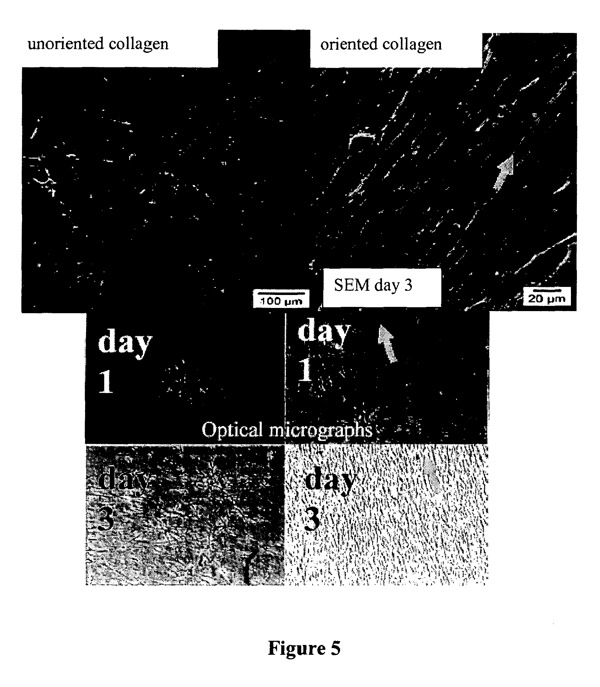

*[Figure 5]* Keratocytes were seeded at the same density on to both unoriented and unidirectionally oriented collagen gels which were either at the original concentration of 2 mg/ml or had undergone dehydration and rehydration with growth medium prior to cell seeding. The cells on the unoriented gel remained randomly distributed after three days while on the oriented support the cells were uniformly aligned along the collagen fibril direction. The cell density was higher in the oriented substrate suggesting that proliferation is more rapid on this support. Figure 5 was obtained using gels that had not undergone any dehydration prior to preparation for SEM study. Similar results were obtained using dried sheets rehydrated with growth medium. Having thus confirmed that dehydration did not impair cell guidance properties dried sheets were used as the starting material for all subsequent experiments.

*[Figure 6]* It is not surprising in the light of passed results that keratocytes align on the surface of oriented collagen (fig. 5). From the point of view of cornea reconstruction it is vital that cells penetrate into the stroma-like scaffold and continue to be steered by contact guidance. Figure 6 shows an example of a three orthogonal lamellae supported on a semi-permeable membrane and seeded with keratocytes. It is clear from this image that the cells both penetrate the matrix and align along the direction of collagen constituting each lamella.

The Inventors have also shown that the growth of epithelial and endothelial cells is also supported by this matrix.

### Discussion

Using magnetic orientation an orthogonal lamellar collagen-based scaffold has been constructed which supports oriented keratocyte growth and penetration *in vitro.* This scaffold can form the basis of an implant that will promote regeneration of damaged cornea in such a way as to give rise to the essential properties inherent to native stroma. There are a number of reasons for optimism. An acellular collagenous matrix of orthogonal lamellae constitutes the primary stroma that serves as the template on which the adult cell-infiltrated stroma organizes (Trelstad and Coulombre, 1971b). It has also been shown (Hu et al., 2005) that the properties of the corneal stroma reconstructed following grafting of an artificial matrix resemble (optical clarity, fibril diameter) those of native stroma. Finally the synthesis of collagen by oriented cells has been shown to be aligned along the cell direction (Wang et al., 2003). Thus keratocytes can infiltrate the acellular scaffold, degrade it while building up a transparent stroma with near native organization.

There are two ways via which it might be possible to construct cell-scaffold composites *in vitro.* It might be possible to drive keratocytes into dilute scaffolds supported on a semi-permeable membrane by flow provided the scaffold is sufficiently porous. Secondly cells could be added to the neutralized collagen solution before exposure to the magnetic field. Although there is evidence suggesting that exposure to a strong magnetic field can impair cell function and viability, the surviving cells appear to recover once the field is removed (Valiron et al., 2005). Prolonged exposure to high fields has also been shown to induce cell orientation (Eguchi et al., 2003;Iwasaka et al., 2003) but this effect is unlikely to be of little consequence in the present context as the samples remain in the field for a relatively short time before being rotated with respect to the field. Both of these methods of creating cell-scaffold composites initially result in rather dilute composites; however, they can be compacted by blotting through the semi-permeable support without exposing the cells to significant osmotic stress. The flow method, if viable, is more likely to have clinical applications as the cell free scaffold could be constructed, transported and stored until use.

*[Improving transparency]* Neither the dilute gels nor the concentrated scaffolds presented here are transparent. It would be better for the patient to have a near transparent implant right from the start. Fortunately oriented scaffolds can be rendered more transparent by adding proteoglycans (PGs). In a separate study the Inventors have observed that transparency is greatly enhanced by the addition of corneal PGs to the neutralized collagen starting solution. In contrast to previous reports, preliminary results suggest that the PGs do not have a significant influence on average fibril diameter but rather increase transparency by break-up of fibril aggregates. Up to about 10% PG/collagen (w/w) the resulting orientation produced by a 7 Tesla field was sufficient to cause contact guidance of surface seeded keratocytes. At the higher PG concentrations required for near transparency this property is lost. High magnetic orientation can only be achieved in conditions favoring ordered cooperativity so to maximize the effective diamagnetic anisotropy. It appears that the presence of PGs reduces this effect, possibly by reducing fibril bundling, which consequently results in lower orientation. The use of a stronger magnetic field could improve the orientation achievable in the presence of higher concentrations of PGs (the magnetic torque would be doubled by increasing the field to just 10 Tesla as it is proportional to the square of the field).

*[Mass production]* For a biomaterial to have the possibility of progressing from laboratory to the clinic, a cost effective manufacturing process is essential. Samples were usually left to orient in the magnet for a total time of about 30 min. However, the optimal field exposure required is probably significantly shorter. The turbidity curve (fig 1) is characterized by a lag phase during which there is no detectable change in turbidity, a growth phase of rapid turbidity change and a plateau region where turbidity is nearly constant. Although the entire process takes at least 15 min, samples need not be maintained in the maximum field throughout fibrillogenesis to obtain maximum orientation. Individual molecules have only a very weakly diamagnetic anisotropy (Worcester, 1978), so high orientation is dependent on the formation of ordered arrays so that the molecular anisotropies add giving rise to an ensemble value sufficient to promote high orientation. During the lag period the solution components interact only weakly hence, under these conditions of poor cooperativity, the magnetic field is ineffective. Thus the presence of the field throughout this phase brings little in the way of improved orientation. During the rapid growth phase collagen molecules interact creating fibrils and fibers, the molecular diamagnetic anisotropies addition giving rise to a relatively large diamagnetic anisotropy and consequently high orientation. As with fibrin (Freyssinet et al., 1983), the degree of alignment is probably "frozen in" about the time the solution becomes a gel. The exact point of gelation is unknown but it probably occurs well before the turbidity has reached its maximum value. These considerations suggest that the sample needs to be in the maximum field only for about 5 min that is the time from the end of the lag-period until the turbidity has reached about half maximum. With the magnet currently in use the maximum area of oriented collagen that can be made simultaneously is about 20 cm².

*[Alternative lamellae]* As fibrin, another frequently used biomaterial, also undergoes magnetic orientation during polymerization, scaffolds of stacked lamellae of oriented fibrin can be created in the way described here for collagen. Laminated collagen-fibrin composites can also be produced in which alternate layers have different physical and bioactive properties.

*[crosslinking]* The above studies were carried out on uncrosslinked collagen. Scaffolds can be crosslinked chemically or using enzymes such as transglutaminase or lysyl oxidase. The latter can probably be used in the presence of cells if desirable. Crosslinking stiffens the scaffold making it easier to manipulate and also alter the rate of matrix degradation.

*[number of lamellae required]* The human stroma contains about 200 lamellae each about 2 µm thick composed of 160 mg/ml collagen.

*[shaping the scaffold]* As the scaffold is initially very dilute it is possible shape it at the time of concentration.

### Conclusions

The Inventors have developed a novel procedure for the production of oriented collagen based scaffolds for use in tissue engineering. The procedure results in a layered structure, within each layer the collagen fibrils are oriented uniaxially while the angle between the fibrils in each successive layer can be chosen at will. Such a twisted plywood structure is seen in a number of connective tissues (for example, cornea, compact bone, intervertebral disc). Collagen based sponges and sheets, which might have other applications in the reconstruction of tendon and muscle, have also been produced. Keratocyte alignment is directed by the collagen orientation both on the surface and within the bulk of the scaffold. All three corneal cell types grow on this scaffold. The scaffold thus has the potential to act as a stand alone matrix or provide the basis for more sophisticated cell supporting constructs.

### REFERENCES

1. Besseau, L., B.Coulomb, C.Lebreton-Decoster, and M.M.Giraud-Guille. 2002. Production of ordered collagen matrices for three-dimensional cell culture. Biomaterials 23:27-36.
2. Boland, E.D., J.A.Matthews, K.J.Pawlowski, D.G.Simpson, G.E.Wnek, and G.L.Bowlin. 2004. Electrospinning collagen and elastin: preliminary vascular tissue engineering. Front Biosci. 9:1422-1432.
3. Ceballos, D., X.Navarro, N.Dubey, G.Wendelschafer-Crabb, W.R.Kennedy, and R.T.Tranquillo. 1999. Magnetically aligned collagen gel filling a collagen nerve guide improves peripheral nerve regeneration. Exp. Neurol. 158:290-300.
4. Dubey, N., P.C.Letourneau, and R.T.Tranquillo. 1999. Guided neurite elongation and schwann cell invasion into magnetically aligned collagen in simulated peripheral nerve regeneration. Exp. Neurol. 158:338-350*.*
5. Eguchi, Y., M.Ogiue-Ikeda, and S.Ueno. 2003. Control of orientation of rat Schwann cells using an 8-T static magnetic field. Neurosci. Lett. 351:130-132.
6. Freyssinet, J.M., J.Torbet, G.Hudry-Clergeon, and G.Maret. 1983. Fibrinogen and fibrin structure and fibrin formation measured by using magnetic orientation. Proc. Natl. Acad. Sci. U. S. A 80:1616-1620.
7. Germain, L., F.A.Auger, E.Grandbois, R.Guignard, M.Giasson, H.Boisjoly, and S.L.Guerin. 1999a. Reconstructed human cornea produced in vitro by tissue engineering. Pathobiology 67:140-147.
8. Griffith, M., R.Osborne, R.Munger, X.Xiong, C.J.Doillon, N.L.Laycock, M.Hakim, Y.Song, and M.A.Watsky. 1999a. Functional human corneal equivalents constructed from cell lines. Science 286:2169-2172.
9. Guido, S. and R.T.Tranquillo. 1993. A methodology for the systematic and quantitative study of cell contact guidance in oriented collagen gels. Correlation of fibroblast orientation and gel birefringence. J. Cell Sci. 105 (Pt 2):317-331.
10. Hu, X., W.Lui, L.Cui, M.Wang, and Y.Cao. 2005. Tissue engineering of nearly transparent corneal stroma. Tissue Eng 11:1710-1717.
11. Iwasaka, M., J.Miyakoshi, and S.Ueno. 2003. Magnetic field effects on assembly pattern of smooth muscle cells. In Vitro Cell Dev. Biol. Anim 39:120-123.
12. Jiang, F., H.Horber, J.Howard, and D.J.Muller. 2004. Assembly of collagen into microribbons: effects of pH and electrolytes. J. Struct. Biol. 148:268-278.
13. Kotani,H., M.Iwasaka, S.Ueno, and A.Curtis. Magnetic orientation of collagen and bone mixture. 87[9], 6191-6193. 2000. AIP.
14. Li, F., M.Griffith, Z.Li, S.Tanodekaew, H.Sheardown, M.Hakim, and D.J.Carlsson. 2005. Recruitment of multiple cell lines by collagen-synthetic copolymer matrices in corneal regeneration. Biomaterials 26:3093-3104.
15. Liu, Y., L.Gan, D.J.Carlsson, P.Fagerholm, N.Lagali, M.A.Watsky, R.Munger, W.G.Hodge, D.Priest, and M.Griffith. 2006. A simple, cross-linked collagen tissue substitute for corneal implantation. Invest Ophthalmol. Vis. Sci. 47:1869-1875.
16. Matthews, J.A., G.E.Wnek, D.G.Simpson, and G.L.Bowlin. 2002. Electrospinning of collagen nanofibers. Biomacromolecules. 3:232-238.
17. Meek, K.M., T.Blamires, G.F.Elliott, T.J.Gyi, and C.Nave. 1987. The organisation of collagen fibrils in the human corneal stroma: a synchrotron X-ray diffraction study. Curr. Eye Res. 6:841-846.
18. Minami, Y., H.Sugihara, and S.Oono. 1993. Reconstruction of cornea in three-dimensional collagen gel matrix culture. Invest Ophthalmol. Vis. Sci. 34:2316-2324.
19. Mosser, G., A.Anglo, C.Helary, Y.Bouligand, and M.M.Giraud-Guille. 2006. Dense tissue-like collagen matrices formed in cell-free conditions. Matrix Biol. 25:3-13*.*
20. Murthy, N.S. 1984. Liquid crystallinity in collagen solutions and magnetic orientation of collagen fibrils. Biopolymers 23:1261-1267.
21. Nishida, K., M.Yamato, Y.Hayashida, K.Watanabe, N.Maeda, H.Watanabe, K.Yamamoto, S.Nagai, A.Kikuchi, Y.Tano, and T.Okano. 2004. Functional bioengineered corneal epithelial sheet grafts from corneal stem cells expanded ex vivo on a temperature-responsive cell culture surface. Transplantation 77:379-385.
22. Pellegrini, G., C.E.Traverso, A.T.Franzi, M.Zingirian, R.Cancedda, and M.De Luca. 1997. Long-term restoration of damaged corneal surfaces with autologous cultivated corneal epithelium. Lancet 349:990-993.
23. Rama, P., S.Bonini, A.Lambiase, O.Golisano, P.Paterna, M.De Luca, and G.Pellegrini. 2001. Autologous fibrin-cultured limbal stem cells permanently restore the corneal surface of patients with total limbal stem cell deficiencyl. Transplantation 72:1478-1485.
24. Schwab, I.R., M.Reyes, and R.R.Isseroff. 2000. Successful transplantation of bioengineered tissue replacements in patients with ocular surface disease. Cornea 19:421-426.
25. Torbet, J. and M.C.Ronziere. 1984. Magnetic alignment of collagen during self-assembly. Biochem. J. 219:1057-1059.
26. Torbet,J., L.Tranqui, and C.Bureau. Magnetic processing of biological and synthetic polymers and crystals. The 3rd International Symposium on Electromagnetic Processing of Materials , 133-137. 2000.
27. Tranquillo, R.T., T.S.Girton, B.A.Bromberek, T.G.Triebes, and D.L.Mooradian. 1996. Magnetically orientated tissue-equivalent tubes: application to a circumferentially orientated media-equivalent. Biomaterials 17:349-357.
28. Trelstad, R.L. and A.J.Coulombre. 1971. Morphogenesis of the collagenous stroma in the chick cornea. J Cell Biol. 50:840-858.
29. Tsai, R.J., L.M.Li, and J.K.Chen. 2000. Reconstruction of damaged corneas by transplantation of autologous limbal epithelial cells. N. Engl. J. Med. 343:86-93.
30. Valiron, O., L.Peris, G.Rikken, A.Schweitzer, Y.Saoudi, C.Remy, and D.Job. 2005. Cellular disorders induced by high magnetic fields. J. Magn Reson. Imaging 22:334-340.
31. Wang, J.H., F.Jia, T.W.Gilbert, and S.L.Woo. 2003. Cell orientation determines the alignment of cell-produced collagenous matrix. J Biomech. 36:97-102.
32. Worcester, D.L. 1978. Structural origins of diamagnetic anisotropy in proteins. Proc. Natl. Acad. Sci. U. S. A 75:5475-5477.

## Claims

1. A method for the *in vitro* preparation of a synthetic multi-layer structure comprising biopolymer fibers, wherein the biopolymer fibers in each layer are unidirectionally oriented, which comprises the following steps :
a) positioning a holder containing a layer of a biopolymer fiber forming solution in a magnetic field and inducing fiber formation, in order to obtain fibers which are unidirectionally oriented in the layer,
b) introducing a further layer of the biopolymer fiber forming solution in the holder, said further layer being introduced over the layer obtained in step a),
c) positioning the holder in the magnetic field and inducing fiber formation, in order to obtain fibers which are unidirectionally oriented in said further layer,
d) optionally, repeating the preceding steps,
thus obtaining a synthetic multi-layer structure.

2. The method according to claim 1, wherein the fiber orientation in at least one layer differs from that in at least one of its superior and/or inferior layer according to an angle alpha, and wherein said differential orientation results from changing by a rotation according to the angle alpha the position of the holder of step c) relative to the position of the holder of step a).

3. The method according to claim 2, wherein the fiber orientation in each layer differs from that in at least one of its superior and/or inferior layer according to the angle alpha.

4. The method according to anyone of claims 1 to 3, wherein the biopolymer fibers are collagen fibers, fibrin fibers, or a mixture thereof.

5. The method according to claim 4, wherein the collagen fibers are type I and/or type V collagen fibers.

6. The method according to claim 4 or 5, wherein the collagen fiber formation is induced by neutralising and heating the collagen fiber forming solution.

7. The method according to claim 6, wherein the neutralisation of the solution is made prior to the heating step, thus allowing to introduce the neutralised biopolymer fiber forming solution in the holder to form one layer, and the heating step is made in the presence of the magnetic field, in order to obtain fibers which are unidirectionally oriented in the layer.

8. The method according to anyone of claims 1 to 7, wherein the synthetic multi-layer structure further comprises proteoglycans.

9. The method according to anyone of claims 1 to 8, wherein the synthetic multi-layer structure further comprises covalent cross-linkages obtained using chemical compounds such as 1-ethyl-3-(3-dimethylaminopropyl) carbodiimide hydrochloride (EDC) or N-hydroxysuccinimide ester (NHS).

10. The method according to anyone of claims 1 to 9, wherein the synthetic multi-layer structure further comprises covalent cross-linkages obtained using enzymes such as transglutaminase or lysyl oxidase.

11. The method according to anyone of claims 1 to 10, wherein the biopolymer fibers are recombinant biopolymer fibers, advantagesouly human recombinant biopolymer fibers.

12. The method according to anyone of claims 1 to 11, wherein the synthetic multi-layer structure is dehydrated by evaporation or blotting using a semi-permeable support.

13. The method according to anyone of claims 1 to 12, wherein the synthetic multi-layer structure is lyophilized.

14. The method according to anyone of claims 1 to 13, wherein the magnetic field is a static magnetic field.

15. The method according to anyone of claims 1 to 14, wherein the angle alpha is comprised between 30° and 150°.

16. The method according to claim 15, wherein the angle alpha is 90°, thus obtaining a synthetic orthogonal multi-layer structure.

17. A synthetic multi-layer structure comprising biopolymer fibers, wherein the biopolymer fibers in each layer are unidirectionally oriented, said structure being obtainable by the method according to anyone of claims 1 to 16.

18. A method for the *in vitro* preparation of a biological tissue-like multi-layer structure which comprises the preparation of a synthetic multi-layer structure according to the method of anyone of claims 1 to 16, and the inoculation of cells therein.

19. The method according to claim 18, wherein the cells are mammalian cells.

20. The method according to claim 18 or 19, wherein the biological tissue-like multi-layer structure is a multi-layer collagen tissue-like structure.

21. The method according to claim 20, wherein the multi-layer collagen tissue-like structure is a multi-layer collagen tissue-like cornea, such as corneal stroma, hemi-cornea or complete cornea.

22. The method according to claim 21, wherein the cells are keratocytes, endothelial cells, epithelial cells or a combination thereof.

23. The method according to anyone of claims 20 to 22, wherein the cell inoculation is made by adding the cells to the neutralised collagen fiber forming solution, and/or by adding the cells to the multi-layer collagen tissue-like structure.

24. A biological tissue-like multi-layer structure comprising the synthetic multi-layer structure according to claim 17 and cells inoculated therein.

25. The biological tissue-like multi-layer structure according to claim 24, wherein the cells are mammalian cells.

26. The biological tissue-like multi-layer structure according to claim 24 or 25, which is a multi-layer collagen tissue-like structure.

27. The biological tissue-like multi-layer structure according to claim 26, wherein the multi-layer collagen tissue-like structure is an orthogonal multi-layer collagen tissue-like cornea, such as a corneal stroma, a hemi-cornea or a complete cornea.

28. The biological tissue-like multi-layer structure according to claim 27, wherein the cells are keratocytes, endothelial cells, epithelial cells or a combination thereof.

29. Use of the synthetic multi-layer structure according to claim 17 for the preparation of a biological tissue-like multi-layer structure.

30. The biological tissue-like multi-layer structure according to anyone of claims 24 to 28 for use as a medicament.

31. Use of the biological tissue-like multi-layer structure according to anyone of claims 24 to 28 as a model for biological testing, such as pharmacotoxicity testing.

32. Use of the orthogonal multi-layer collagen tissue-like cornea according to claim 27 or 28 for the preparation of a medicament for preventing or treating a damaged corneal tissue in a subject, such as corneal stroma, hemi-cornea, complete cornea or a combination thereof.
